**Europäisches Patentamt**

**European Patent Office**

**Office européen des brevets**

(11) Veröffentlichungsnummer: **0 034 862 B1**

# EUROPÄISCHE PATENTSCHRIFT

(45) Veröffentlichungstag der Patentschrift:
31.07.85

(51) Int. Cl.⁴: **A 61 B 6/02**

(21) Anmeldenummer: **81200181.6**

(22) Anmeldetag: **17.02.81**

(54) Vorrichtung zur Erzeugung von Schichtbildern eines dreidimensionalen Objektes mit Hilfe von Überlagerungszonogrammen.

(30) Priorität: **23.02.80 DE 3006828**

(43) Veröffentlichungstag der Anmeldung:
**02.09.81 Patentblatt 81/35**

(45) Bekanntmachung des Hinweises auf die Patenterteilung:
**31.07.85 Patentblatt 85/31**

(84) Benannte Vertragsstaaten:
**DE FR GB IT SE**

(56) Entgegenhaltungen:
**DE - A - 1 914 393**
**FR - A - 1 336 114**
**FR - A - 2 306 463**
**FR - A - 2 341 149**
**GB - A - 2 016 855**
**US - A - 3 818 220**

(73) Patentinhaber: **Philips Patentverwaltung GmbH, Billstrasse 80, D-2000 Hamburg 28 (DE)**
(84) Benannte Vertragsstaaten: **DE**

(73) Patentinhaber: **N.V. Philips' Gloeilampenfabrieken, Groenewoudseweg 1, NL-5621 BA Eindhoven (NL)**
(84) Benannte Vertragsstaaten: **FR GB IT SE**

(72) Erfinder: **Curth, Claus-Peter, Brandenburger Strasse 16B, D-2900 Oldenburg (DE)**
Erfinder: **Tiemens, Ulf, Ludorffstrasse 62, D-5860 Iserlohn (DE)**
Erfinder: **Klotz, Erhard, Seekamp 110, D-2083 Halstenbek (DE)**

(74) Vertreter: **Hartmann, Heinrich, Dipl.-Ing. et al, Philips Patentverwaltung GmbH Billstrasse 80 Postfach 10 51 49, D-2000 Hamburg 28 (DE)**

**Beschreibung**

Die Erfindung betrifft eine Vorrichtung zur schichtweisen Darstellung eines dreidimensionalen Objektes, mit einer Gruppe von in einer Strahlenquellenebene liegenden Strahlenquellen zur Durchstrahlung eines auf einem Aufnahmetisch liegenden Objektes, und mit einem unterhalb des Objektes liegenden Aufzeichnungsträger zur Aufzeichnung eines aus einzelnen Perspektivbildern bestehenden kodierten Bildes des Objektes.

Eine derartige Vorrichtung ist bereits aus der DE-OS 2 605 497 bekannt. Mit ihr wird ein Objekt mittels einer Mehrfachstrahlquelle, die aus mehreren in einer Ebene liegenden einzelnen Strahlenquellen besteht, unter verschiedenen Perspektiven durchstrahlt und auf einem unterhalb des Objektes befindlichen Aufzeichnungsträger als kodiertes Überlagerungsbild aufgezeichnet. Eine Dekodierung des Überlagerungsbildes zur Darstellung von Schichtbildern des Objektes kann dann anschließend z. B. mit Hilfe des in der DE-OS 2 414 322 beschriebenen Dekodierverfahrens erfolgen.

Nachteilig bei der Herstellung der kodierten Bilder mittels dieser Vorrichtung ist jedoch, daß durch die überlagerte Abbildung von Zentralprojektionen unterschiedlicher Perspektive Überlagerungsartefakte im dekodierten Bild durch Objektstrukturen, z. B. Knochen, entstehen, die nicht im interessierenden Schichtbereich des Objektes liegen, wodurch die Qualität der dekodierten Schichtbilder vermindert wird. Ferner ist von Nachteil, daß alle sich überlagernden Zentralprojektionen mit einem relativ großen Kontrastumfang abgebildet werden, so daß in der von einer Zentralprojektion schon stark belichteten Filmfläche die Objektinformation verloren geht, die von anderen Perspektiven auf die gleiche Fläche projiziert wird. Der Kontrastumfang eines Schichtbildes bzw. Zonogramms ist wesentlich geringer als bei einer Zentralprojektion.

Aufgabe der Erfindung ist es daher, eine Vorrichtung zur Erzeugung von Schichtbildern anzugeben, mit deren Hilfe Artefakte im dekodierten Schichtbild aufgrund von außerhalb der rekonstruierten Objektschicht liegenden Objektdetails vermindert werden.

Die Aufgabe wird gemäß der Erfindung dadurch gelöst, daß die Strahlenquellengruppe und der Aufzeichnungsträger in zueinander parallelen Ebenen relativ zum ruhenden Objekt derart gegensinnig verschiebbar angeordnet sind, daß sich die Zentralstrahlen der jeweiligen Strahlenquellenbündel bei allen Stellungen der Strahlenquellengruppe in einem Punkt des Objektes schneiden.

Die Aufgabe der Erfindung kann ferner auch dadurch gelöst werden, daß das Objekt und der Aufzeichnungsträger in zur Strahlenquellenebene parallelen Ebenen relativ zur ruhenden Strahlenquellengruppe derart gleichsinnig verschiebbar angeordnet sind, daß sich die Zentralstrahlen der jeweiligen Strahlenquellenbündel bei allen Stellungen des Objektes in einem Punkt des Objektes schneiden.

Während der Aufnahme eines kodierten Bildes mit Hilfe der erfindungsgemäßen Vorrichtung wird also von dem durchstrahlten Objekt nur ein vorher bestimmter zonographischer Bereich, z. B. eine Objektschicht von einigen mm bis cm Dicke, scharf abgebildet, während alle außerhalb der Objektschicht liegenden Objektdetails verwischt aufgezeichnet werden. Hierdurch wird erreicht, daß im dekodierten Bild die Artefaktbildung aufgrund der außerhalb der interessierenden Objektschicht liegenden Objektstrukturen erheblich vermindert wird. Der scharf abgebildete Schichtbereich steht dann zur Dekodierung mehrerer Schichtbilder des Objektes mit den an sich bekannten Dekodierverfahren zur Verfügung.

Vorteilhaft ist hierbei die Kontrastwiedergabe der Schicht. Da die außerhalb der Objektschicht liegenden Objektdetails schon bei der Aufnahme verwischt werden, stören sie in der Schichtdarstellung bei dem Dekodiervorgang nur noch geringfügig. Man hat im Prinzip eine doppelte Verwischung der nicht zur Objektschicht gehörenden Objektdetails. Die erste bei der zonographischen Aufzeichnung, die zweite bei dem Dekodiervorgang. Dadurch wird ein Kontrastgewinn erreicht.

Weitere Vorteile bringt die Aufzeichnung von überlagerten Zonogrammen dadurch, daß

1. der Film nicht unnötig von Objektstrukturen geschwärzt wird, die nicht im interessierenden Bereich liegen, und
2. für die eigentliche Objektinformation eine weniger stark durchmodulierte Filmfläche ausgenutzt werden kann.

Nach einer vorteilhaften Weiterbildung der Erfindung ist als Aufzeichnungsträger eine Simultankassette zur gleichzeitigen Aufnahme mehrerer untereinanderliegender Schichten des Objektes vorgesehen, wodurch erreicht wird, daß mit nur einem Aufzeichnungsvorgang mehrere Gebiete des Objektes dekodierbar sind.

Als mögliches Anwendungsgebiet der erfindungsgemäßen Vorrichtung ist z. B. die Knochentomographie zu sehen. Besonders gut kann die Vorrichtung auch bei selektiven Schichtuntersuchungen z. B. der Gehörgänge, der Orbita oder der Wirbelsäule, d. h. bei jenen Objekten, deren Tiefenausdehnung mit wenigen aneinandergelegten zonographischen Objektbereichen erfaßt werden kann, eingesetzt werden.

Die Zeichnung stellt ein Ausführungsbeispiel der Erfindung dar. Es zeigt

Fig. 1 eine Skizze zur Erläuterung der Schichtdicke bei einem Zonogramm,

Fig. 2 eine Tomosynthese-Vorrichtung zur Erzeugung zonographischer Schichtaufnahmen, und

Fig. 3 eine Tomosynthese-Vorrichtung mit einer Simultankassette zur gleichzeitigen Erzeugung mehrerer zonographischer Schichtaufnahmen.

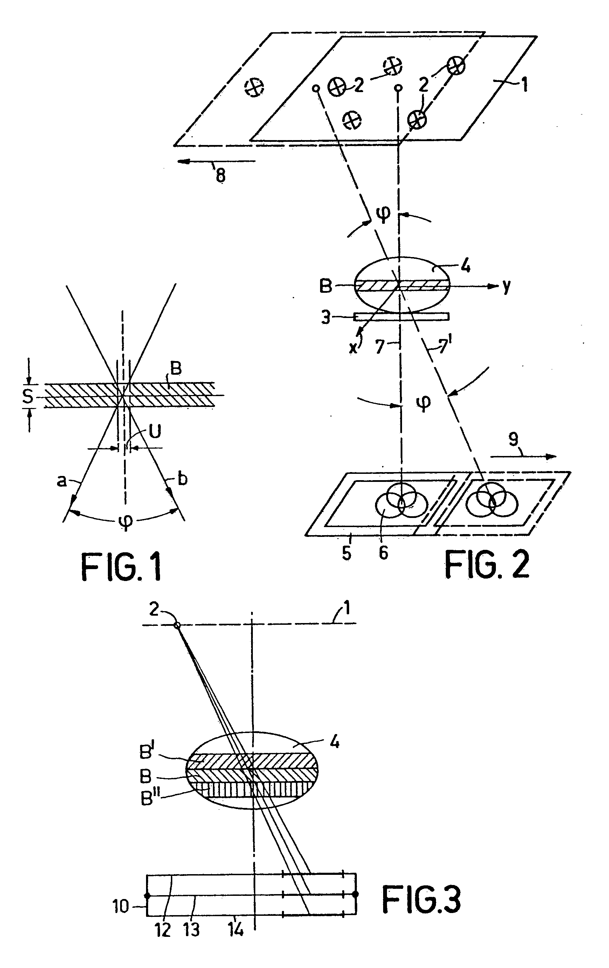

In der Fig. 1 ist eine Skizze zur Erläuterung der Schichtdicke bei einem Zonogramm dargestellt. Die Schichtdicke S ist dabei abhängig von dem Schichtwinkel $\varphi$ zwischen zwei Linien a, b, durch die z. B. jeweils die Zentralstrahlen der Strahlenbündel einer verschiebbaren Strahlenquelle angegeben sind, und der zulässigen bzw. für das entsprechende Anwendungsgebiet vertretbaren Gesamtunschärfe U. Alle Objektdetails, die sich während der Aufnahme des Zonogramms innerhalb des von der schraffierten Fläche dargestellten Bereichs B befinden, werden scharf abgebildet. Die Schärfe der Abbildung ist dabei von der Gesamtunschärfe U abhängig, die sich aus dem Schichtwinkel $\varphi$ und der Schichtdicke S ergibt. Alle ober- und unterhalb des schraffierten Bereichs B liegenden Objektstrukturen werden mit einer größeren Gesamtunschärfe und daher verwischt abgebildet, so daß die durch derartige Objektstrukturen verursachten Artefakte erheblich reduziert werden.

Fig. 2 zeigt eine Tomosynthese-Vorrichtung zur Erzeugung von zonographischen Schichtaufnahmen. Sie besitzt mehrere in einer Strahlenquellenebene 1 liegende Strahlenquellen 2, die entsprechend einer vorgegebenen Punktverteilung (siehe z. B. DE-OS 2 830 186) angeordnet sind. Die Zahl der Strahlenquellen (Röntgenröhren) kann z. B. 25 oder mehr betragen, während in Fig. 2 lediglich drei Strahlenquellen dargestellt sind.

Natürlich kann auch eine einzelne Strahlenquelle benutzt werden, die nacheinander an die verschiedenen Positionen zur Durchstrahlung des Objektes geführt wird.

Unterhalb der Strahlenquellen 2 befindet sich ein auf einem Aufnahmetisch 3 liegendes Objekt 4, das von den Strahlenbündeln der Strahlenquellen 2 derart durchstrahlt wird, daß auf einem unterhalb des Objektes 4 liegenden Aufzeichnungsträger 5, z. B. einem Röntgenfilm, die einzelnen Perspektivbilder des Objektes 4 als kodiertes Überlagerungsbild 6 aufgezeichnet werden. Die Richtung unter der die Strahlenquellen 2 das Objekt 4 durchstrahlen, kann beispielsweise entlang einer senkrecht durch den Bereich B des Objektes 4 hindurchtretenden Linie 7 verlaufen, zu der die Strahlenquelle 1 und der ebene Aufzeichnungsträger 5 ebenfalls senkrecht liegen. Sowohl die Strahlenquellen 2, die z. B. in einem gemeinsamen Tank zu einer Mehrfachstrahlenquelle zusammengefaßt sind, als auch der Aufzeichnungsträger 5 sind in ihren jeweiligen Ebenen verschiebbar angeordnet. Zur Erzeugung einer zonographischen Aufnahme des Objektes 4 können somit die Strahlenquellen 2 unter Beibehaltung ihres gegenseitigen Abstandes und der Aufzeichnungsträger 5 in jeweils entgegengesetzten (gegensinnigen) Richtungen 8, 9 entlang z. B. gerader Bahnen um gleiche Winkel $\varphi$ relativ zur ersten Strahlenrichtung 7 ausgelenkt werden, so daß das Objekt 4 unter einer weiteren Strahlenrichtung 7' durchstrahlt wird, wobei der Schnittpunkt der Strahlenrichtung 7' mit der Strahlenrichtung 7 innerhalb des Objektbereichs B liegt. Die Auslenkung bzw. Verschiebung erfolgt also so, daß sich alle Verbindungsgeraden zwischen jeweils einer Strahlenquelle und dem Zentrum des Aufzeichnungsträgers 5 bei allen Stellungen der Strahlenquellengruppe relativ zum ruhenden Objekt in einem Punkt (Objektpunkt) schneiden. Zu diesem Zweck werden die einzelnen Strahlenquellen 2 in den jeweiligen Positionen so verstellt, daß ihre Strahlenbündel immer auf das Objekt 4 gerichtet sind. Die Strahlenquellen 2 können dabei entlang ihrer Bahnen kontinuierlich oder an bestimmten Stellen kurzzeitig eingeschaltet werden.

Natürlich kann statt der Verschiebung von Strahlenquellengruppe 2 und Aufzeichnungsträger 5 auch eine gleichsinnige Verschiebung des Objektes 4 und des Aufzeichnungsträgers 5 in entsprechender, oben dargestellter Weise parallel zur ruhenden Strahlenquellengruppe 2 erfolgen. Dabei bleiben die einzelnen Strahlenbündel immer auf das Objekt ausgerichtet.

Eine andere Möglichkeit zur Erzeugung von zonographischen Aufnahmen besteht darin, die Strahlenquellengruppe 2 und den Aufzeichnungsträger 5 in ihren parallel zueinander liegenden Ebenen auf Kreisbahnen in entgegengesetzter Richtung umlaufen zu lassen, durch deren Mittelpunkt z. B. die senkrecht zur Schichtebene B liegende Linie 7 verläuft, und relativ zu der die Strahlenquellengruppe 2 und der Aufzeichnungsträger 5 um gleiche Winkel $\varphi$ ausgelenkt sind. Auch hier erfolgt die Bewegung so, daß sich alle Verbindungsgeraden zwischen jeweils einer Strahlenquelle und dem Zentrum des Aufzeichnungsträgers 5 bei allen Stellungen der Strahlenquellengruppe relativ zum ruhenden Objekt in einem Punkt (Objektpunkt) schneiden. Dabei wird die Strahlenquellengruppe 2 entlang der Kreisbahn so bewegt, daß bei einer Draufsicht senkrecht auf die Strahlenquellenebene 1 die Verbindungslinien zwischen den Einzelstrahlenquellen die Koordinatenachsen x, y eines ebenen, parallel zur Strahlenquellenebene liegenden und mit dem Objekt 4 fest verbundenen Koordinatensystems immer unter gleichen Winkeln schneiden. Entsprechend läuft der Aufzeichnungsträger 5 auf einer Kreisbahn um.

Selbstverständlich ist es auch in diesem Fall möglich, die Strahlenquellengruppe 2 fest zu positionieren, und dafür das Objekt 4 bzw. den Aufnahmetisch 3 und den Aufzeichnungsträger 5 in gleicher Richtung in entsprechender Weise auf Kreisbahnen umlaufen zu lassen. Natürlich können ebenso andere gekrümmte, ebene Bahnen, z. B. Ellipsenbahnen durchlaufen werden. Die Strahlenquellen können auch hier wieder kontinuierlich oder kurzzeitig an bestimmten Punkten ihrer Bahn eingeschaltet werden.

In Fig. 3 ist dargestellt, wie von dem Objekt 4 mit Hilfe einer Simultankassette 10, in der z. B. drei im Abstand übereinander liegende Röntgenfilme 12, 13, 14 angeordnet sind, drei aufeinander liegende Schichten B', B, B'' zonographisch aufgezeichnet werden. Dabei wird auf dem Film 12 die Schicht B', auf dem Film 13 die Schicht B und auf dem Film 14 die Schicht B'' aufgezeichnet. Die auf den Röntgenfilmen 12, 13, 14 befindlichen Aufnahmen stellen gleichzeitig kodierte Bilder dar, von denen jeweils eines zur Dekodierung von weiteren, innerhalb der auf dem entsprechenden Röntgenfilm scharf abgebildeten Körperschicht liegenden Schichtbildern herangezogen werden kann. Der Übersicht wegen ist in Fig. 3 nur eine einzige Strahlenquelle der Strahlenquellengruppe 2 dargestellt.

## Patentansprüche

1. Vorrichtung zur schichtweisen Darstellung eines dreidimensionalen Objektes, mit einer Gruppe von in einer Strahlenquellenebene liegenden Strahlenquellen zur Durchstrahlung eines auf einem Aufnahmetisch liegenden Objektes, und mit einem unterhalb des Objektes liegenden Aufzeichnungsträger zur Aufzeichnung eines aus einzelnen Perspektivbildern bestehenden kodierten Bildes des Objektes, dadurch gekennzeichnet, daß die Strahlenquellengruppe (2) und der Aufzeichnungsträger (5) in zueinander parallelen Ebenen relativ zum ruhenden Objekt (4) derart gegensinnig verschiebbar angeordnet sind, daß sich die Zentralstrahlen der jeweiligen Strahlenquellenbündel bei allen Stellungen der Strahlenquellengruppe in einem Punkt des Objektes schneiden.

2. Vorrichtung zur schichtweisen Darstellung eines dreidimensionalen Objektes, mit einer Gruppe von in einer Strahlenquellenebene liegenden Strahlenquellen zur Durchstrahlung eines auf einem Aufnahmetisch liegenden Objektes, und mit einem unterhalb des Objektes liegenden Aufzeichnungsträger zur Aufzeichnung eines aus einzelnen Perspektivbildern bestehenden kodierten Bildes des Objektes, dadurch gekennzeichnet, daß das Objekt (4) und der Aufzeichnungsträger (5) in zur Strahlenquellenebene (1) parallelen Ebenen relativ zur ruhenden Strahlenquellengruppe (2) derart gleichsinnig verschiebbar angeordnet sind, daß sich die Zentralstrahlen der jeweiligen Strahlenquellenbündel bei allen Stellungen des Objektes in einem Punkt des Objektes schneiden.

3. Vorrichtung nach Anspruch 1 oder 2, dadurch gekennzeichnet, daß die Strahlenquellengruppe (2) und der Aufzeichnungsträger (5) bzw. das Objekt (4) und der Aufzeichnungsträger auf geraden Bahnen verschiebbar sind.

4. Vorrichtung nach Anspruch 1 oder 2, dadurch gekennzeichnet, daß die Strahlenquellengruppe (2) und der Aufzeichnungsträger (5) bzw. das Objekt (4) und der Aufzeichnungsträger auf gekrümmten, vorzugsweise Kreisbahnen derart verschiebbar angeordnet sind, daß bei einer Draufsicht auf die Strahlenquellenebene (1) die Verbindungslinien zwischen den Strahlenquellen die Koordinatenachsen (x, y) eines ebenen, parallel zur Strahlenquellenebene (1) liegenden und mit dem Objekt fest verbundenen Koordinatensystem immer unter gleichen Winkeln schneiden.

5. Vorrichtung nach einem oder mehreren der Ansprüche 1 bis 4, dadurch gekennzeichnet, daß als Aufzeichnungsträger (5) eine Simultankassette zur gleichzeitigen Aufnahme mehrerer untereinander liegender Schichten des Objektes (4) vorgesehen ist.

## Claims

1. A device for layer-wise imaging of a three-dimensional object, comprising a group of radiation sources which are situated in a radiation source plane in order to irradiate an object which is arranged on an examination table, and also comprising a record carrier which is situated underneath the object in order to record an encoded image of the object which consists of separate perspective images, characterized in that the radiation source group (2) and the record carrier (5) are arranged to slide in opposite directions mutually parallel planes relative to a stationary object (4) so that central rays of the respective radiation source beams intersect one another in one point of the object for all positions of the radiation source group.

2. A device for the layer-wise imaging of a three-dimensional object, comprising a group of radiation sources which are arranged in a radiation source plane in order to irradiate an object which is arranged on an examination table, and also comprising a record carrier which is situated underneath the object in order to record an encoded image of the object which consists of separate perspective images, characterized in that the object (4) and the record carrier (5) arre arranged to slide in planes parallel to the radiation source plane in the same direction relative to the stationary radiation source group (2) so that the central rays of the respective radiation source beams intersect one another in one point of the object in all positions of the object.

3. A device as claimed in claim 1 or 2, characterized in that the radiation source group (2) and the record carrier (5) or the object (4) and the record carrier can be slid along straight paths.

4. A device as claimed in claim 1 or 2, characterized in that the radiation source group (2) and the record carrier (5) or the object (4) and the record carrier are arranged to be slidable along curved, preferably circular paths so that, in a plan view of the radiation source plane (1), the connecting lines between the radiation sources intersect the coordinate axes (x, y) of a flat coordinate system, situated parallel to the radiation source plane (1) and permanently associated with the object, always at the same angle.

5. A device as claimed in one or more of the claims 1 to 4, characterized in that for the record carrier (5) use is made of a multiple-film cassette for the simultaneous recording of a plurality of layers of the object (4) which are situated one underneath the other.

**Revendications**

1. Dispositif pour la représentation tomographique d'un objet tridimensionnel, comportant un groupe de sources de rayonnement disposées dans un plan de sources de rayonnement pour que leurs rayons traversent un objet posé sur une table de support, et un support d'enregistrement disposé en dessous de l'objet pour enregistrer une image codée de l'objet faite de vues en perspective individuelles, caractérisé en ce que le groupe de sources de rayonnement (2) et le support d'enregistrement (5) sont montés dans des plans parallèles l'un à l'autre, déplaçables en sens opposés par rapport à l'objet (4) immobile de telle sorte les rayons centraux des divers faisceaux de sources de rayonnement se coupent en un point de l'objet pour toutes les positions du groupe de sources de rayonnement.

2. Dispositif pour la représentation tomographique d'un objet tridimensionnel, comportant un groupe de sources de rayonnement disposées dans un plan de sources de rayonnement pour que leurs rayons traversent un objet posé sur une table de support, et un support d'enregistrement disposé en dessous de l'objet pour enregistrer une image codée de l'objet faite de vues en perspective individuelles, caractérisé en ce que l'objet (4) et le support d'enregistrement (5) sont montés dans des plans parallèles au plan des sources de rayonnement (1), déplaçables dans le même sens par rapport au groupe de sources de rayonnement (2) immobile de telle sorte que les rayons centraux des divers faisceaux des sources de rayonnement se coupent en un point de l'objet pour toutes les positions de l'objet.

3. Dispositif suivant la revendication 1 ou 2, caractérisé en ce que le groupe de sources de rayonnement (2) et le support d'enregistrement (5) ou l'objet (4) ainsi que le support d'enregistrement peuvent être déplacés suivant des trajets rectilignes.

4. Dispositif suivant la revendication 1 ou 2, caractérisé en ce que le groupe de sources de rayonnement (2) et le support d'enregistrement (5) ou l'objet (4) et le support d'enregistrement sont montés déplaçables suivant des trajets courbes, de préférence circulaires, d'une manière telle que , dans le cas d'une vue en plan du plan des sources de rayonnement (1), les lignes de liaison entre les sources de rayonnement coupent toujours sous des angles égaux les axes de coordonnées (x, y) d'un système de coordonnées plan, parallèle au plan des sources de rayonnement (1) et lié de manière fixe à l'objet.

5. Dispositif suivant l'une quelconque des revendications 1 ou 4, caractérisé en ce qu'une cassette simultanée destinée à enregistrer simultanément plusieurs couches de l'objet (4) disposées l'une en dessous de l'autre est prévue à titre de support d'enregistrement (5).

FIG. 1

FIG. 2

FIG. 3